# EUROPEAN PATENT SPECIFICATION

(11) **EP 2 531 254 B1**
(45) Date of publication and mention of the grant of the patent: **13.05.2015**
(21) Application number: 11704145.9
(22) Date of filing: 04.02.2011
(51) Int. Cl.: A61M 39/02, A61M 39/04, A61F 5/00

(54) **IMPLANTABLE SUBCUTANEOUS ACCESS PORT**
IMPLANTIERBARER SUBKUTANER ZUGANGSPORT
ORIFICE D'ACCÈS SOUS CUTANÉ IMPLANTABLE

(30) Priority: 17.11.2010 US 948703; 02.11.2010 US 409449 P; 02.11.2010 US 409440 P; 05.02.2010 US 301910 P
(43) Date of publication of application: 12.12.2012
(73) Proprietor: APOLLO ENDOSURGERY, INC., Austin, TX 78746 (US)
(72) Inventor: RAVEN, Joseph S., Goleta, California 93117 (US); TRILOKEKAR, Nikhil, Goleta, California 93117 (US); CHITRE, Kaustubh, Goletta, California 93117 (US)
(74) Representative: Hoffmann Eitle
(86) International application number: PCT/US2011/023709
(87) International publication number: WO 2011/097451

(56) References cited:
- EP-A1- 2 070 494
- US-A- 5 133 753
- US-A1- 2008 039 772
- US-A1- 2009 099 538
- US-B2- 6 997 914

## Description

### RELATED APPLICATIONS

This application claims priority to and the benefit of U.S. Provisional Patent Application No. 61/301,910, entitled "TISSUE EXPANDER" filed on February 5, 2010; U.S. Provisional Patent Application No. 61/409,440, entitled "TISSUE EXPANDER" filed on November 5, 2010; and U.S. Provisional Patent Application No. 61/409,449, entitled "PUNCTURE RESISTANT COMPOSITE MATERIALS" filed on November 2, 2010.

### Field

The present invention generally relates to medical systems and apparatus and uses thereof for treating obesity and/or obesity-related diseases, and more specifically, relates to an implantable subcutaneous access port.

### BACKGROUND

Adjustable gastric banding apparatus have provided an effective and substantially less invasive alternative to gastric bypass surgery and other conventional surgical weight loss procedures. Despite the positive outcomes of invasive weight loss procedures, such as gastric bypass surgery, it has been recognized that sustained weight loss can be achieved through a laparoscopically-placed gastric band, for example, the LAP-BAND® (Allergan, Inc., Irvine, CA) gastric band or the LAP-BAND AP® (Allergan, Inc., Irvine, CA) gastric band. Generally, gastric bands are placed about the fundus, or cardia, or esophageal junction, of a patient's upper stomach forming a stoma that restricts food's passage into a lower portion of the stomach. When the stoma is of an appropriate size that is restricted by a gastric band, the food held in the upper portion of the stomach may provide a feeling of satiety or fullness that discourages overeating. Unlike gastric bypass procedures, gastric band apparatus are reversible and require no permanent modification to the gastrointestinal tract. An example of a gastric banding system is disclosed in Roslin, et al., U.S. Patent Pub. No. 2006/0235448.

Existing gastric bands periodically require adjustments to maintain an effective constriction about the stomach, to account for changes in the stomach tissue, reduction of fat or other factors causing movement and/or size change of the stomach. Some attempts have been made to allow for such adjustment of gastric bands. For example, hydraulic gastric bands utilize a fluid such as saline to fill an inflatable portion of the gastric band using a subcutaneous injection access port. Adjustments to the amount of inflation may be made by injecting or extracting the fluid through the patient's skin into or out of the injection access port, which then directs the fluid into or out of the inflatable portion of the gastric band.

Current access ports are typically configured to include a flat-surfaced septum, a rigid housing, and a substantially flat base. The housing forms a rim around the septum to expose a circular surface area of the septum.

U.S. Pat. No. 6,997,914 issued to Smith and Fowler describes an implantable access port comprising a self-sealing, penetrable septum secured onto the base of the access port by a retainer ring, wherein the base and the retainer ring are preferably composed of a biocompatible material, such as electro-polished stainless steel or hard material such as titanium, and a dome-shaped reservoir inside the port.

U.S. Pat. No. 5,041,098 issued to Loiterman et al. describes an implantable access port comprising a self-sealing, penetrable septum affixed to a housing, wherein the housing is preferably formed of hard materials such as titanium or surgical grade steel and includes a lip which lines the peripheral portion of the septum.

U.S. Pat. No. 4,190,040 issued to Schulte describes a subcutaneous injection housing that has a generally dome-shaped sealant chamber which can reseal needle punctures in the housing and has a generally flat base.

U.S. Pat. No. 4,543,088 issued to Bootman and Yamamoto describes a resealable puncture housing for surgical implantation having a generally dome-shaped wall, which comprises an upper wall and a side wall, wherein the outer surface of the upper wall has a substantially flat surface and the side wall is generally straight and vertical.

The access ports currently on the market suffer from a known series of drawbacks, including patient discomfort due to the shape or rigidity of the subcutaneously implanted access port, and patients' aesthetic concerns about the appearance of the access port. A rigid access port may press against, and damage, portions of the patient's body during the natural body motion of the patient. In addition, the rigid construction of access ports currently on the market may serve as an irritant beneath the patient's skin. In addition, the rim, housing, and septum configuration of many access ports on the market may produce an aesthetic concern for the patient. As the patient loses weight over time, the access port will gradually become more visible through the patient's skin. The rim of the septum may exhibit a mechanical or ring-like shape beneath the patient's skin, which may embarrass the patient.

In addition, access ports currently on the market may be difficult to locate subcutaneously by palpation or other means, and may be difficult to penetrate with syringe needles. Physicians may not be able to easily detect the flat upper surface of an access port currently on the market, located beneath many layers of fat. In addition, once the physician detects the access port, the physician must attempt to pass a syringe through the layers of fat, and into a septum, which may comprise a relatively small proportion of the total outer surface area of the housing. The physician may miss the septum entirely and may contact internal organs of the patient, causing great pain. In addition, the physician may contact the tubing leading from the access port, causing the tubing to rupture, and requiring surgery to mend the tube, or to replace the entire gastric banding system.

Accordingly, it is desirable to develop a gastric banding system, and an implantable access port designed to remedy the deficiencies of access ports currently on the market.
US 2009/0099538 A1 discloses an access port for a gastroplasty band for the treatment of obesity having a housing provided with a puncture zone able to be transpierced by a hollow needle for injecting fluid into and/or tapping fluid from a chamber provided inside the housing. The housing is provided with a duct for communication with the gastroplasty band. The housing includes a perforated skeleton forming the structure of the housing and imparting to the housing its overall shape.

### SUMMARY

Generally described herein is a gastric banding system, including an implantable access port having a substantially ellipsoid shape. The access port is pliable and smooth, to increase the comfort of the access port to the patient, and to increase the aesthetic effect of the access port. The ellipsoid shape provides a large needle penetrable surface area for the access port, and enhances the ability of a physician to detect the access port through tactile means.

The access port includes a housing comprising an ellipsoid-shaped shell defining an internal fluid reservoir. The shell has an anterior side and a posterior side, the anterior side being the side of the housing facing the patient's outer skin layer, and the posterior side being the opposite side of the housing, facing away from the patient's outer skin layer. The outer surface of the anterior side of the housing may be made entirely from a needle penetrable and self-sealing material. A portion of the posterior side of the housing may be made from a needle penetrable and self-sealing material, and a portion of the posterior side of the housing may be made from a needle-resistant material. The needle penetrable and self-sealing material of the anterior side and/or posterior side preferably comprises a pliant material, to provide a degree of compliance for the housing.

A mesh layer is included in a portion of the anterior side of the housing and/or in a portion of the posterior side of the housing. The mesh layer of the anterior side of the housing may be contiguous with the mesh layer of the posterior side of the housing. The mesh layer serves to enhance the self-sealing properties of the housing, by constraining the materials of the housing, to prevent fluid from leaking from the reservoir.

A layer of needle resistant material may cover a portion of the posterior side of the housing, or the entire posterior side of the housing. The needle resistant material may also cover a portion of the anterior side of the housing. The needle resistant material may be positioned adjacent to the fluid reservoir, to reduce the possibility of a syringe needle passing through the housing, once it enters the reservoir.

An anchor member is fixed to a portion of the housing, preferably the posterior side of the housing. The anchor member may comprise a mesh member made of a mesh material, being biocompatible. The mesh material may be capable of integrating with the local body tissue of the patient. The mesh material may be sutured or tacked to the patient's body. The anchor member may have a skirt-like, sheet-like, or disk-like shape, and may extend out from the housing at a diameter being larger than the diameter of the housing. In one embodiment, the mesh material of the anchor member may be contiguous with the mesh layer of the posterior side of the housing and/or the mesh layer of the anterior side of the housing.

A connecting tube extends from a conduit in the housing. The connecting tube transfers fluid between the gastric band and the fluid reservoir. The connecting tube may extend from the posterior side of the housing, and, in one embodiment, may pass through a portion of the needle resistant material, and/or the anchor member. The connecting tube may extend from the posterior side of the housing at an angle, preferably an acute angle. The angle reduces erosion of local tissue and organs caused by the connecting tube. In addition, if the tube passes beneath the needle resistant material and/or the anchor member, then the needle resistant material and/or anchor member may shield the connecting tube from puncture by an incoming syringe needle. In one embodiment, the anchor member may be integrated with a ring, which offers further protection for the connecting tube.

The implantable access port is designed to overcome known deficiencies of access ports currently on the market, including pain associated with the access port, aesthetic concerns associated with the access port, difficulty in locating the access port, difficulty in locating a penetrable surface of the access port, and difficulty penetrating the access port with a syringe.

The access port is preferably made of soft, or pliant, materials that allow the housing of the access port to flex or deform when a force is exerted against the housing. The pliant nature of the access port may reduce the pain associated with a rigid device placed within a patient's body. In addition, the surface of the access port housing has a substantially curved profile, which reduces the number of sharp angles or edges of the access port. Fewer angles or edges in the patient's body may reduce damage caused by the access port.

The access port also has a smooth, curved outer shell, which produces a more organic appearance than the rim, housing, and septum combination, of known gastric bands. The organic appearance may increase the aesthetics of the access port, if the access port is visible through the skin of the patient. The outline of the access port may appear invisible, or less visible than access ports currently on the market.

The access port also reduces the difficulty in locating the access port through tactile means. The access port has an ellipsoid shape, which provides a distinct volume and structure, which may be easily identified and detected by a physician.

The access port also increases the total penetrable surface area of the housing beyond the septum size of access ports currently on the market. The outer surface of the housing effectively comprises the septum of the housing, and is needle penetrable from various incident angles. Currently, access ports on the market commonly employ a substantially flat-surfaced septum as the facet of the port available for percutaneous needle insertion. A rigid housing forms an impenetrable rim around the septum. In these access ports, the proportion of the port accessible for percutaneous needle insertion is limited to a central, circular region on top of the housing. The total needle penetrable surface of the present invention's access port may be between two times and three times larger than access ports currently on the market (e.g., access ports currently on the market may have a septum size of approximately 0.17 square inches, the present invention contemplates a septum size of approximately 0.45 square inches).

In addition, access ports currently on the market may be available for percutaneous top-down entry of a syringe needle, only if the penetrable septum is facing, or is parallel to the surface of the skin. As a result, if such an access port inverts or otherwise changes its usual orientation such that the exposed septum surface is no longer facing or is parallel to the surface of the skin, a needle cannot enter the port to deliver fluids. The access port is penetrable from multiple angles, including angles along the sides of the housing, which allow the access port to remain functional even if it varies its orientation within the patient's body.

The access port of the present invention, in at least one embodiment, increases the protection offered by the access port for the connecting tube. The connecting tube may pass beneath the housing, the needle resistant material, and/or the anchor member, to allow these structures to further protect the tube from puncture by incident syringe needles. The connecting tube may also pass underneath a ring of the anchor member, which further protects the tube from puncture by incident syringe needles.

### BRIEF DESCRIPTION OF THE DRAWINGS

The exemplary embodiments of the present invention will be described in conjunction with the accompanying drawing FIGS. in which like numerals denote like elements and:
FIG. 1 illustrates a perspective view of a gastric banding system according to an embodiment of the present invention;
FIG. 2 illustrates a cross-sectional view of an access port according to an embodiment of the present invention;
FIG. 2A illustrates a close-up cross-sectional view of a portion of the anterior side of the access port housing according to an embodiment of the present invention;
FIG. 3 illustrates a perspective view of an access port displaying a x-axis, a y-axis, and a z-axis of the access port housing according to an embodiment of the present invention;
FIG. 4 illustrates a cross-sectional view of an access port according to an embodiment of the present invention;
FIG. 5 illustrates a cross-sectional view of an access port according to an embodiment of the present invention;
FIG. 6 illustrates an exploded perspective view of an access port according to an embodiment of the present invention;
FIG. 7 illustrates a cross-sectional view of an access port according to an embodiment of the present invention; and
FIG. 8 illustrates a side view of an access port according to an embodiment of the present invention.

### DETAILED DESCRIPTION

The present invention generally provides a gastric banding system, including an implantable access port having a substantially ellipsoid shape. The access port is pliable and smooth, to increase the comfort of the access port to the patient, and to increase the aesthetic effect of the access port. The ellipsoid shape provides a large needle penetrable surface area for the access port, and enhances the ability of a physician to detect the access port through tactile means.

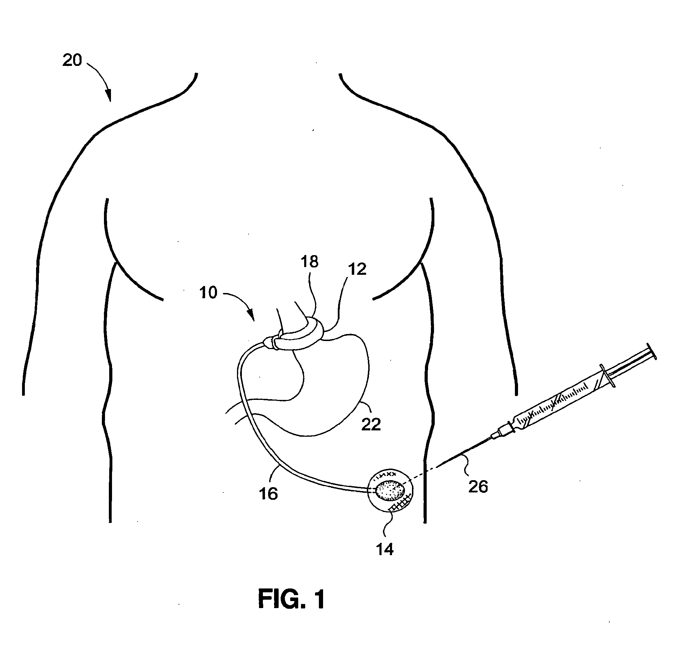

FIG. 1 illustrates a gastric banding system **10** contemplated for use in the present invention, comprising a gastric band **12,** an access port **14,** and a connecting tube **16.** The gastric band **12** may include a lumen, an inflatable portion, or inflatable bladder **18,** which is capable of being filled with fluid. The connecting tube **16** couples the bladder **18** to the access port **14** and supplies fluid to the inflatable bladder **18.**

The gastric band **12** forms a loop around a portion of a patient's, or individual's **20** stomach **22.** The portion of the stomach **22** receiving the gastric band **12** may comprise the cardia, the fundus, or the esophageal junction of an individual's **20** stomach **22.** The loop constricts a portion of the individual's **20** stomach **22** to form a stoma, which restricts the flow of food entering the lower portion of the stomach **22** when the individual **20** eats. The restricted flow of food promotes a more rapid production of satiety signals during times of food consumption, than are normally produced without the restricted flow of food. The increased satiety signals hopefully cause the individual **20** to feel full more quickly, and cause the individual **20** to reduce food intake. The reduced food intake hopefully causes the individual **20** to lose weight over time.

It is preferable if the degree of constriction applied by the gastric band **12** to the stomach **22** is variable. A variable degree of constriction is preferred because the biological characteristics of an individual's **20** stomach **22** may vary over time. For example, the individual's **20** stomach **22** may increase or decrease in size, requiring an appropriately larger or smaller degree of constriction. In addition, the degree of constriction may need to be varied if the individual **20** is not losing weight in response to the gastric band treatment, or if the weight loss is not at a desired level. If the individual **20** is not responding appropriately to the gastric band therapy, the gastric band **12** may need to be adjusted, to increase the degree of constriction, and to further decrease the flow of food to the lower portion of the stomach. In addition, it is preferable if the size of the gastric band **12** itself is variable, to accommodate the unique biological characteristics of different patients, for example, patients having a smaller or larger sized stomach.

To accommodate a varying degree of constriction, or varying gastric band **12** size, the gastric band **12** may comprise an adjustable gastric band **12,** or comprise a gastric band **12** having an adjustable diameter. The gastric band **12** may be adjusted by adjusting the amount of fluid in the bladder **18.** The bladder **18** may extend around the portion of the stomach **22** to be constricted, and may have a variable size, functioning as an inflatable cuff placed around the stomach **22.** An increased bladder **18** size may increase the degree of constriction applied to the stomach **22,** and a decreased bladder **18** size may decrease the degree of constriction. Accordingly, an increased amount of fluid in the bladder **18** may increase the degree of constriction, and a decreased amount of fluid in the bladder **18** may decrease the degree of constriction.

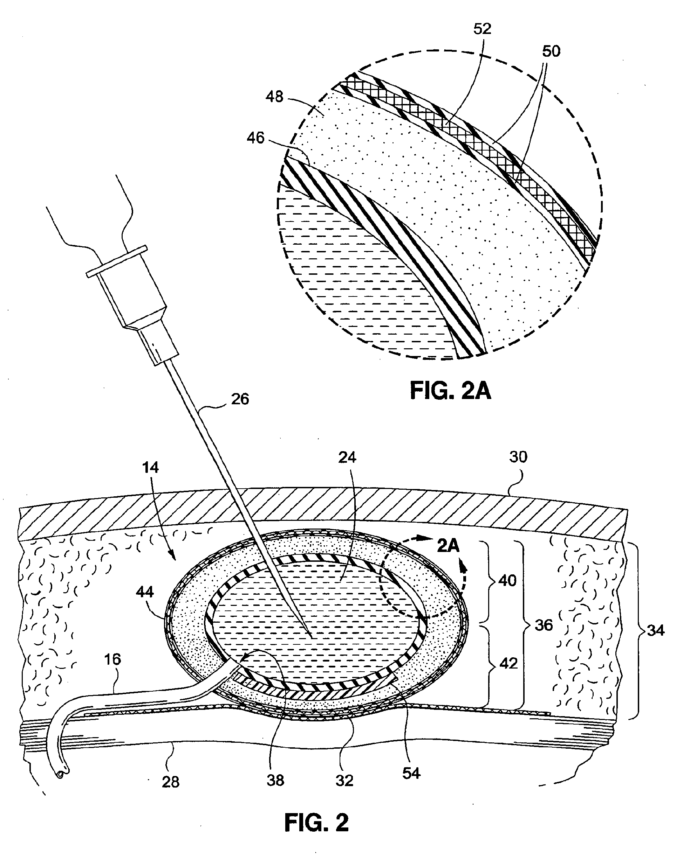

The amount of fluid in the bladder **18** may be controlled via the access port **14.** The access port **14** is preferably positioned subcutaneously within the individual's **20** body and is preferably secured to a firm layer of tissue, for example, the individual's **20** muscle wall. The access port **14** may include an internal fluid reservoir **24,** as shown in FIG. 2, in fluid communication with the bladder **18** of the gastric band **12,** through the connecting tube **16.** If more fluid is injected into the reservoir **24,** then the bladder **18** increases in size, and the degree of constriction is increased. If fluid is removed from the reservoir **24,** then the bladder **18** decreases in size, and the degree of constriction decreases. A physician accesses the access port **14** through the skin of the patient, to vary the amount of fluid in the fluid reservoir **24.** The physician inserts a syringe **26** through the skin of the patient, to penetrate the access port **14,** and add or remove fluid from the fluid reservoir **24.** The access port **14** is therefore preferably positioned near the surface of the individual's **20** skin, to allow a physician to more easily access the access port **14** with the syringe **26.**

FIG. 2 illustrates an embodiment of the access port **14** shown in FIG. 1. The access port **14** is shown coupled to the muscle wall **28** of the individual, beneath the individual's skin **30.** An anchor member **32** connects the access port **14** to the muscle wall **28.** A fat layer **34** surrounds the access port **14.**

The access port **14** includes a housing **36** that has a substantially ellipsoidal shape, and defines a reservoir **24** within the interior of the housing **36.** A fluid conduit **38** is positioned within the housing **36** and connects the fluid reservoir **24** to the connecting tube **16.** The connecting tube **16** is coupled to the gastric band **12,** as shown in FIG. 1.

The housing **36** defines a shell around the fluid reservoir **24.** The shell is formed by the combination of two half-shells, one half-shell being defined by an anterior side **40** of the housing **36** and other half-shell being defined by a posterior side **42** of the housing **36.** The anterior side **40** includes the portion of the access port **14** positioned towards the individual's skin **30,** and above the midline **44** of the access port **14,** which represents a maximal width of the access port **14,** formed due to the ellipsoidal shape of the access port **14.** The anterior side **40** is the top-half of the housing **36,** and has a substantially dome-like, or half-dome shape. Once the access port **14** is implanted beneath the skin **30** of a patient, the anterior side **40** of the housing **36** comprises a penetrable surface that faces, and is positioned directly beneath, the patch of skin **30** through which a syringe **26** needle can be inserted, to inject fluid into the access port **14.** The posterior side **42** includes the portion of the access port **14** positioned towards the individual's muscle wall **28,** and below the midline **44** of the access port **14.** The posterior side **42** is the bottom-half of the housing **36,** and has a substantially dome-like or half-dome shape.

The anterior side **40** of the housing **36** is made from at least one material being needle penetrable and self-sealing. The anterior side **40** of the housing **36** may include a single layer of material, or multiple layers of materials placed adjacent to each other. The single layer of material, or multiple layers of material are preferably resilient, but firm enough to enable detection of the access port beneath the skin by palpation. A layer of material may include silicones of scale (A) durometer 65 +/- 35 and scale (D) durometer 50 +/- 20, with a thickness greater than 0.080 inches. The layer or layers of material are preferably needle penetrable and self-sealing because the anterior side **40** must sustain repeated, periodic insertion and withdrawal of needles without leakage of fluid from inside the access port **14.** The layer or layers are preferably made of a biocompatible material, that is nontoxic and not harmful to mammals. Silicone elastomer, such as a dimethyl or dimethyl-diphenyl silicone elastomer, may be used in a single layer or multiple layers of the anterior side **40.**

Referring to FIG. 2A, the anterior side **40** of the housing **36** may comprise multiple layers of material, including an interior layer **46,** an intermediate silicone gel layer **48,** an exterior layer **50,** and a mesh layer **52.** The interior layer **46** defines an outer boundary of the fluid reservoir **24,** and may extend around and encompass the fluid reservoir **24.** The interior layer **46** of the anterior side **40** may have a half-dome shape, similar to the shape of the anterior side **40** of the housing **36.** The interior layer **46** of the anterior side **40** may extend entirely around the anterior side **40** of the fluid reservoir **24.** The interior layer **46** is preferably made from an elastomeric material. The elastomeric material is needle penetrable and self-sealing, to withstand repeated and periodic insertion and withdrawal of needles, without causing fluid to leak from the fluid reservoir **24.**

The silicone gel layer **48** extends over, adjacent to, and around the interior layer **46,** encircling the interior layer **46.** The silicone gel layer **48** comprises a soft and pliable layer of silicone gel extending around the interior layer **46.** The silicone gel layer **48** may aid the interior layer **46** to prevent fluid from leaving the reservoir **24,** by providing a barrier layer to the fluid. In addition, the soft and malleable nature of the gel layer **48** also increases the overall softness and pliability of the housing **36,** and the access port **14.**

The exterior layer **50** may comprise two layers of elastomeric material placed adjacent to, or on both sides of, a mesh layer **52.** The exterior layer **50** defines the outer boundary, or outer surface of the housing **36.** The anterior side **40** portion of the exterior layer **50** may have a shape that defines the half-dome shape of the anterior side **40.** The elastomeric materials comprising the two layers of the exterior layer **50** may have similar properties as the material comprising the interior layer **46,** namely, the elastomeric materials of the exterior layer **50** may be needle penetrable and self sealing.

The embedded mesh layer **52** may be integrated, or embedded, within the exterior layer **50.** The embedded mesh layer **52** enhances the resealability of the exterior layer **50,** and adds a degree of structure to the housing **36,** allowing the housing **36** to retain an ellipsoid shape after manipulation by a physician, insertion of needles, and contact with other elements of the surrounding environment (e.g., other parts of the interior of the patient's body, or forces exerted from outside the patient's body). The mesh layer **52** may act in combination with the gel layer **48** to enhance the resealability of the housing **36** shell. For example, after a void is created in the shell by a needle used to introduce fluid into the reservoir **24,** the gel layer **48** prevents the fluid from having a direct path to the exterior of the housing **36.** The mesh layer **52** enhances this property of the gel layer **48** by physically constraining the gel from expansion under pressure exerted by the fluid within the reservoir **24.** The material used in the mesh layer **52** may comprise polyethylene terephthalate, polypropylene, polyamide, polyethylene, and combinations thereof. The material used in the mesh layer **52** may also comprise any other material capable of producing an equivalent result.

Referring back to FIG. 2, the posterior side **42** of the housing **36** may also have a similar layered composition as the anterior side **40.** For example, the posterior side **42** may include an interior layer **46** being made from an elastomeric material. The interior layer **46** may extend entirely around the posterior side **42** of the fluid reservoir **24,** notwithstanding the position of the conduit **38** extending through the interior layer **46.** The elastomeric material of the posterior side's **42** interior layer **46** may be needle penetrable and self-sealing. In addition, the posterior side **42** may include a silicone gel layer **48** positioned adjacent to the interior layer **46.** The posterior side **42** may also include an exterior layer **50** with an embedded mesh **52.** The exterior layer **50** may have a half-dome shape that defines the half-dome shape of the posterior side **42.** The interior layer **46,** gel layer **48,** exterior layer **50,** and mesh **52** of the posterior side **42** may have a similar configuration and composition as the interior layer **46,** gel layer **48,** exterior layer **50,** and mesh **52** of the anterior side **40.** The elastomeric material of the posterior side's **42** exterior layer **50** may be needle penetrable and self-sealing.

The posterior side **42** may also include one or more layers of needle-resistant material **54** extending around a portion of the fluid reservoir **24.** The single layer or multiple layers of needle-resistant material **54** may be positioned outside the fluid reservoir **24** adjacent to the interior layer **46** (as shown in FIG. 2). The needle-resistant material **54** may be bonded to the interior layer **46,** within the shell of the housing **36.** The needle-resistant material **54** may have a curved shape, to contour to the shape of the interior layer **46,** or the fluid reservoir **24.** In one embodiment, the needle-resistant material **54** may be bonded to the exterior layer **50.** In one embodiment, the entirety of the posterior side **42,** or a portion of the posterior side **42** may comprise only a single layer or multiple layers of needle-resistant material.

The layer of needle-resistant material **54** may comprise any biocompatible reinforced fabric, or textile. For example, the needle-resistant material **54** may comprise any suitable biocompatible polymer available in the art, such as KEVLAR®, with sufficient thickness to resist puncturing. Other materials may include specialty material, specialty woven material, and reinforced fabrics. Examples of other needle-resistant materials that may be used include polyethylene, polypropylene, polyimide, thermoplastic polyurethanes, higher durometer silicone, acrylonitrile butadiene styrene, and the like. It is contemplated that various other materials may be utilized that produce an equivalent result.

In one embodiment, the anterior side **40** and the posterior side **42** may be constructed of the same layers of material, although the posterior side **42** additionally contains a layer of needle-resistant material **54.** In one embodiment, the anterior side **40** may comprise a layer or layers of material being different than a layer or layers of material of the posterior side **42.** For example, the posterior side **42** may comprise only a layer of needle-resistant material **54** and the anterior side **40** may comprise the exterior layer **50** containing mesh **52,** the intermediate layer of silicone gel **48,** and the interior layer **46** of elastomeric material. In addition, in one embodiment, the mesh material used in the mesh layer **52** of the anterior side **40** and posterior side **42** may be different. In addition, in one embodiment, the mesh material used in the mesh layer **52** of the anterior side **40** and the posterior side **42** may be made of the same material, and contiguous. In addition, in one embodiment, the shell of the housing **36** may comprise a dual-lumen, gel-filled silicone, without an integrated mesh layer **52.** In addition, in one embodiment, the entire shell of the housing **36** may comprise a singular silicone layer. Various combinations of materials and layers may be used in the anterior side **40** and the posterior side **42** to produce a desired result.

The anterior side **40** and the posterior side **42** may be manufactured separately, then sealed together to form the shell of the housing **36.** The anterior side **40** and the posterior side **42** may be sealed together through ultrasonic welding, or other equivalent techniques. In one embodiment, the entire housing **36,** including the anterior side **40** and the posterior side **42,** may be constructed as a whole, and may not require separate sides **40, 42** to be fused together. The housing **36** is preferably formed as a whole when the anterior side **40** and the posterior side **42** are made from similar material layers, notwithstanding the additional needle-resistant material **54** present in the posterior side **42.** To manufacture the housing **36** as a whole, a dipping method may be used. The dipping method may be used to form an embodiment of the housing **36** including an exterior layer **50** having an embedded mesh **52,** an intermediate silicone gel layer **48,** an interior layer **46,** and a layer of needle resistant material **54,** as shown in FIG. 2.

The dipping method generally comprises making the exterior layer **50** of the housing **36** by dipping two or more layers of silicone-based elastomer over a mandrel in the desired shape of the housing **36,** for example, a substantially ellipsoid shape. A pre-fabricated mesh is placed over the formed elastomer layers, followed by two or more dips of the silicone-based elastomer. The mesh is then embedded between layers of elastomer, as shown in FIG. 2A. The entire assembly of the exterior layer **50** is then heated in an oven at a temperature and for a period of time suitable to cure the silicone. Post-curing, the exterior layer **50** of the housing is removed from the mandrel. The interior layer **46** is formed in a similar manner, by dipping layers of silicone-based elastomer over a mandrel.

The interior layer **46** is placed inside the exterior layer **50,** and the exterior layer **50** and the interior layer **46** are vulcanized. A compartment is formed between the exterior layer **50** and the interior layer **46.** The assembly of the exterior layer **50** and the interior layer **46** is then mounted back on a mandrel. The size the mandrel may be the same as the one used for the fabrication of the interior layer **46** or slightly larger, as a slightly larger mandrel would result in a laterally stressed inner shell with potentially enhanced sealing properties. The compartment formed between the exterior layer **50** and the interior layer **46** is then filled with silicone gel. This may be accomplished using any suitable method known to those skilled in the art. The silicone gel between the exterior layer **50** and the interior layer **46** forms the intermediate gel layer **48.** After the compartment is filled with gel, the assembly of the exterior layer **50,** the interior layer **46,** and the gel layer **48** is exposed to heat in an oven for a suitable period of time. Before sealing the housing **36** with a patch, a needle-resistant material **54** is inserted and bonded to the interior layer **46** and/or the exterior layer **50.**

The housing **36** encloses a space, or cavity, that serves as the fluid reservoir **24.** The interior layer **46** extends around and encompasses the fluid reservoir **24.** The fluid reservoir **24** may have the same shape as the housing **36** (e.g., an ellipsoid shape). The reservoir **24** may also have varied equivalent shapes capable of retaining a measure of fluid. The pressure inside the reservoir **24** may range from 0 mmHg to 800 mmHg, although it is contemplated this pressure range may be varied as desired.

In the embodiment shown in FIG. 2, the anchor member **32** comprises a mesh member that is directly joined to the posterior side **42** of the housing **36.** The anchor member **32** attaches the access port **14** to body tissue. The anchor member **32** may be secured to the posterior side **42** of the housing **36** by ultrasonic welding or other suitable means. The mesh anchor member **32** preferably has a substantially symmetrical shape, such as a square or a circle. Only a portion of the mesh anchor member **32,** preferably a central portion, is joined to the posterior side **42.** The portion of the mesh anchor member **32** that is not joined to the posterior side is available for attachment to subcutaneous tissue (e.g., the muscle wall **28)** via sutures or surgical tacks, or other equivalent means. The mesh anchor member **32** may also connect to subcutaneous tissue by merely placing the anchor member **32** on the desired fixing point of the tissue, and allowing local tissue to integrate with the mesh, fixing the anchor member **32** in place. The mesh may also be flexible, to allow the physician to manipulate the mesh during implantation. In addition, a flexible mesh may allow the anchor member **32** to be contoured to fit along varied shapes of the patient's body to which it is fixed. The mesh material is preferably biocompatable to allow the anchor member **32** to integrate with the tissue of the patient's body, and strengthen the body's bond to the access port **14,** and increase the body's biological acceptance of the access port **14** (e.g., reducing the chance of infection and other signs of biological rejection of the port). The mesh may be made from a material comprising polyethylene terephthalate, polypropylene, polyamide, polyethylene, and combinations thereof. The mesh material of the anchor member **32** may be the same material used as the embedded mesh **52** of the anterior side **40,** or the posterior side **42,** or may comprise a different mesh material.

The conduit **38** comprises a channel, or passageway through the housing **36,** that allows fluid to enter or exit the fluid reservoir **24** from or to the exterior of the housing **36,** respectively. The conduit **38** may be positioned near the posterior side **42** of the housing **36,** as shown in FIG. 2, or may be positioned in varied locations along the fluid reservoir **24** that may provide an equivalent result (e.g., near a lower portion of the anterior side **40** of the housing **36).** The conduit **38** allows fluid within the reservoir **24** to pass through the layer, or layers of material surrounding the reservoir **24,** and to pass to the gastric band **12,** as shown in FIG. 1. The conduit **38** is preferably positioned near the posterior side **42** of the housing **36** to allow the connecting tube **16** to extend from the conduit **38** in a direction away from a syringe **26** inserted by a physician.

The connecting tube **16** connects to the conduit **38,** and fluidly couples the reservoir **24** to the gastric band **12** (as shown in FIG. 1). As shown in FIG. 2, the connecting tube **16** may extend from the posterior side **42** of the housing **36.** The connecting tube **16** extends in a direction away from the anterior side **40** of the housing **36,** to reduce the possibility that the connecting tube **16** is punctured by an incoming syringe **26.** The connecting tube **16** may extend along the surface of the anchor member **32** and then pass through a portion of the patient's body (e.g., the muscle wall **28)** to connect to the gastric band **12.** The connecting tube **16** may be made from silicone elastomer, or the equivalent. The connecting tube **16** may be of any length suitable to connect the access port **14** to the gastric band **12,** depending on the desired placement of the access port **14** and the gastric band **12.** For example, the connecting tube **16** may have a length of five inches, if the access port **14** is placed along the abdominal muscle sheath of the patient.

In operation, the access port **14** receives a syringe **26** inserted through the skin **30** of the patient. The syringe **26** penetrates the layer, or multiple layers, of the anterior side **40** of the housing **36** until the syringe **26** enters the fluid reservoir **24.** The syringe **26** may also enter the housing **36** through the posterior side **42** of the housing **36,** if the syringe **26** does not contact the needle resistant material **54.** The syringe **26** deposits or removes fluid from the fluid reservoir **24.** When the syringe **26** is withdrawn from the housing **36,** the composition of the anterior side **40** of the housing **36,** or possibly the posterior side **42** of the housing **36,** prevents fluid from exiting the reservoir **24.**

The construction of the access port **14** provides benefits that aid both the physician and patient in the use and comfort of the access port **14.** For example, the layer of needle-resistant material **54** is positioned along the posterior side **42** of the housing **36** to prevent the syringe **26** needle from penetrating through the housing **36.** Thus, after the physician locates the housing **36,** the physician may fully insert the syringe **26** needle into the housing **36** without great concern that the syringe **26** will pass through the housing **36.** In addition, the access port **14** also provides the benefit that the outer layers of the housing **36** extend entirely around the fluid reservoir **24.** The outer layers of the housing **36** provide a large penetrable surface for the physician to contact with the syringe **26.** In the embodiment shown in FIG. 2, the entirety of the anterior side **40** of the housing **36** is needle penetrable. In addition, approximately 60% of the entire outer surface of the housing **36** is needle penetrable. The large penetrable surface area allows the physician to insert a needle into the access port **14** from a variety of different angles and make contact with the fluid reservoir **24.** A greater penetrable surface area improves accessibility of the access port **14** for percutaneous needle insertion, which decreases the likelihood of multiple attempts at needle insertion and the accompanying pain and discomfort to patients. The accessible needle surface area of the access port **14** is preferably at least 0.45 square inches. It is contemplated this surface area may be increased or decreased to produce varied desired results.

The access port **14** provides other benefits, including the pliant and resilient composition of the housing **36.** The elastomeric materials preferably composing the interior layer **46,** and the exterior layer **50,** are pliable and may deform when a force is exerted against the housing **36.** The housing **36** is preferably firm enough to palpate when pressurized with fluid, yet is not a completely rigid structure. In addition, the silicone gel layer **48** provides a deformable cushion layer between the interior layer **46** and the exterior layer **50.** The housing **36** therefore is not a stiff, or hard, device implanted within in the patient's body. The pliable nature of the housing **36** may increase total comfort of the device for the patient, as a rigid port within the patient's body may irritate local tissue surrounding the port **14.** The pliable housing **36** may also reduce the chance of injury to the patient when forces are exerted on the housing **36,** as the housing **36** will absorb a measure of the force exerted.

Another benefit of the embodiment shown in FIG. 2 includes the ellipsoid shape of the housing **36.** The ellipsoid shape produces a large needle penetrable surface area for the physician to contact with a syringe **26** needle. The syringe **26** may not only be inserted from directly above the access port **14,** but may also enter the access port **14** through a variety of incident angles. The variety of insertion points may be beneficial if the access port **14** twists or moves relative to the desired orientation within the body. In obesity treatments, movement of the access port **14** is a common concern, as the patient will hopefully undergo rapid weight loss, which may vary the position of the access port **14** relative to the surface of the patient's skin **30.** A variety of entry points, positioned along both the anterior side **40** and the posterior side **42** of the housing **36** increases the total number of successful entry points for the access port **14.**

The ellipsoid shape also provides the benefit that the housing **36** forms a bead-like object that can be easily identified by the physician through tactile means. A physician can run his or her hand along the surface of the patient's skin **30,** and may detect the midline **44** of the access port **14** connecting the anterior side **40** and the posterior side **42** of the housing **36.** The ellipsoid shape thus provides a unique, identifiable, structural presence that is more easily detectable within the patient's body than merely a half-dome or half-shell embodiment. The ellipsoid shape is thus beneficial when the housing **36** may be positioned within a thick fat layer **34** surrounding the access port **14.** When used for obesity treatments, the three-dimensional ellipsoid shape enhances the possibility of detection for patients with large fat deposits.

In addition, the ellipsoid shape also forms a smooth contour shape beneath the patient's skin, which may increase the cosmetic, or aesthetic, effect of the housing **36.** If the outline, or contour, of the housing **36** is visible through the surface of the patient's skin, the smooth contour produced by the ellipsoid shape may be more visually appealing than the outline produced by a rigid, angled, access port.

The use of a mesh anchor member **32** also provides benefits, including an enhanced degree of biocompatibility between the access port **14** and the patient's body. An expansive mesh provides a broad area for attaching the access port **14** to the patient's body through sutures, surgical tacks, or the like. Moreover, during post-operation healing, growing scar tissue becomes intertwined with the mesh, which enhances the security of the housing **36** to subcutaneous tissue. The more stable or secure the attachment, the less likely that the access port **14** may flip, invert, or dislodge, which lowers the difficulty of accessing the penetrable surface of the access port **14** for fluid injection or withdrawal. A stable attachment spares patients from pain associated with multiple attempts at injection, and reduces the need for reoperation to adjust the access port **14** position.

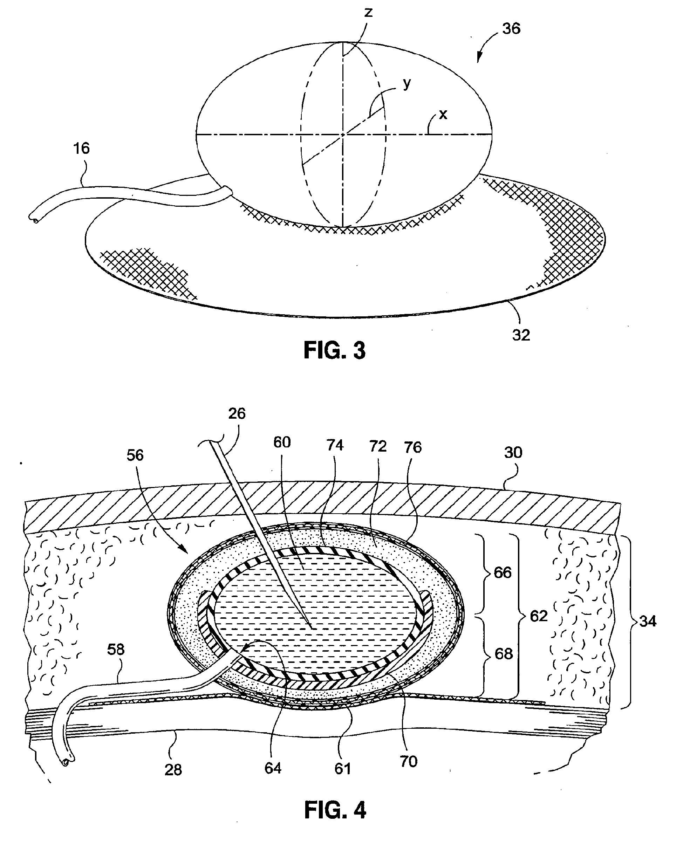

Referring to FIG. 3, the ellipsoidal shape of the housing **36** contemplates, but is not limited to shapes including a sphere and spheroid. Descriptions of ellipsoids typically involve reference to the x, y, and z axes of these structures. FIG. 3 illustrates a diagrammatic three-dimensional view of an embodiment of the housing **36** which shows the x, y, and z axes of the substantially ellipsoid-shaped housing **36.** If the ellipsoid housing **36** were configured as a sphere, the housing **36** would have x, y, and z axes of equal length. If the ellipsoid housing **36** were configured as an oblate spheroid, the x and y axes would have the same length, the length being greater than the length of the z-axis. In one embodiment, each portion of the outer surface of the housing **36** has a curved local gradient, meaning each portion of the outer surface is curved. In one embodiment, only a portion of the outer surface of the housing **36** has a curved local gradient, but the housing **36** otherwise forms a substantially ellipsoid shape. In one embodiment, the anterior side **40** and the posterior side **42** (as defined in FIG. 2) of the housing **36** each have a domed, or half dome shape. In this embodiment, from the perspective of the fluid reservoir, the anterior side **40** and the posterior side **42** each have a convex shape. The housing **36** is not limited to an ellipsoid shape, but may have various non-ellipsoid shapes, for example, a half-dome, or half-shell shape, as desired.

As used in FIG. 3, the z-axis of the housing **36** refers to the height of the housing **36,** or the dimension extending perpendicular to the surface of the muscle wall **28,** as shown in FIG. 2. The x-axis and y-axis are orthogonal axes extending perpendicular to the z-axis, and extend substantially parallel to the surface of the patient's skin **30,** as shown in FIG. 2. A plane formed by the x-axis and y-axis also extends substantially parallel to the surface of the patient's skin **30.** The x-axis and y-axis define a width of the housing **36.** In one embodiment, the width of the housing **36** along the x-axis and the y-axis is approximately equal, and may range between about 0.75 to about 1.75 inches, inclusive. In this embodiment, the height of the housing along the z-axis may range between about 0.60 and about 1.00 inches, inclusive. The defined widths and height of the housing **36** include the outer extent of the material layers comprising the housing **36.** In one embodiment, the width of the housing **36** along the x-axis and the y-axis is equal, and is about 1.25 inches. In this embodiment, the length of the z-axis is about 0.80 inches. In one embodiment, the housing **36** may be shaped such that the width along the x-axis and the y-axis is unequal, and the width along the x-axis is greater than along the z-axis or the y-axis, as shown in FIG. 3. In other embodiments, the length along the axes may be varied as desired, to produce various sizes and shapes of the housing **36.** The housing **36** may include any configuration of ellipsoid shapes, including sphere, or spheroid, or any other desired equivalent shapes that produce smooth curved contours of the housing **36** within the patient's body, or are capable as forming an outer surface for the housing **36.**

FIG. 3 also illustrates the shape of the anchor member **32** in more detail than shown in FIG. 2. The anchor member **32** may extend out from the base of the housing **36** with a diameter being larger than that of the housing **36.** The anchor member **32** may comprise a substantially flat sheet of mesh, that may have a disk-like, or skirt-like shape. The anchor member **32** may also be shaped or contoured to match the shape of the desired fixing point within the patient's body.

FIG. 4 illustrates a layer of needle resistant material **70** extending entirely around the portion of the fluid reservoir **60** on the posterior side **68** of the housing **62,** and partially around the portion of the fluid reservoir **60** on the anterior side **66** of the housing **62.** The needle resistant material **70** extends adjacent to the interior layer **74** of the housing **62,** and between the interior layer **74** and the exterior layer **76.** The needle resistant material **70** may be positioned within the gel layer **72** of the housing **62.** A portion of the needle resistant material **70** includes a hole or passage, to allow the conduit **64** and the connecting tube **58** to pass therethrough. In the embodiment shown in FIG. 4, the total needle penetrable region of the housing **62** comprises approximately 40% of the total surface area of the housing **62.** An increased size of needle resistant material **70** decreases the possibility that a needle passes through the housing **62,** but also decreases the total needle penetrable surface area of the access port **56,** and the housing **62.**

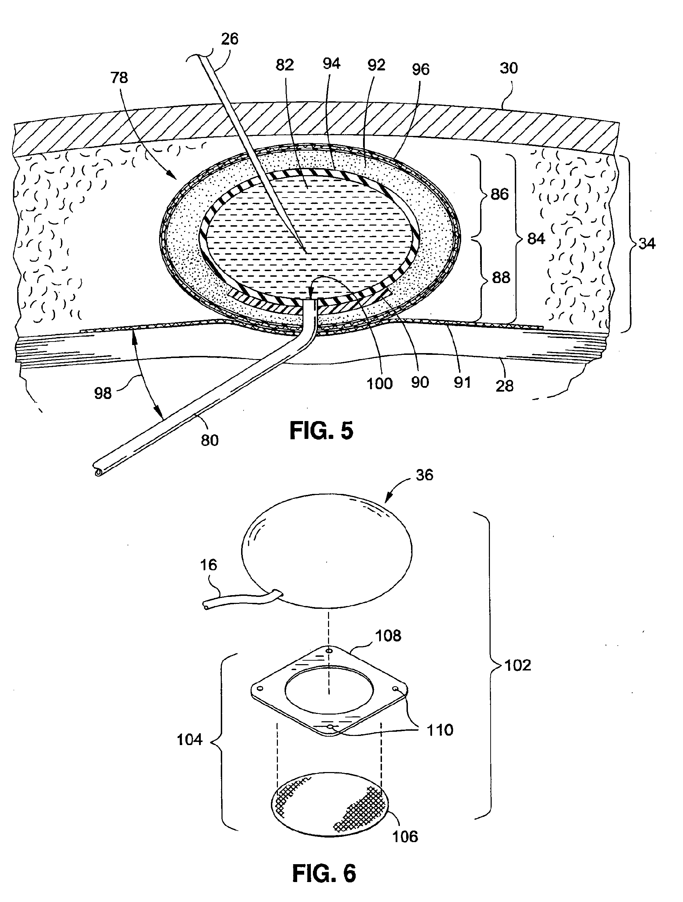

FIG. 5 illustrates a conduit **100** positioned at the lowest point of the fluid reservoir **82,** or equivalently referred to as the lowest height of the fluid reservoir **82,** or equivalently referred to as a point of lowest local surface curvature of the fluid reservoir **82,** or otherwise referred to as the center or apex of the posterior side **88** of the housing **84.** The conduit **100** is positioned at the center of the posterior side **88** of the housing **84** to allow a connecting tube **80** to pass through the center of the posterior side **88** of the housing **84.** The connecting tube **80** extends through the layer of a needle resistant material **90** and through the anchor member **91.** The needle resistant material **90** and the anchor member **91** are configured similarly to the embodiments shown in FIG. 2, aside from having holes, or passages, to accommodate passage of the connecting tube **80.** The conduit **100** may also be positioned at any other equivalent position along the housing **84** that allows the tube **80** to pass through the needle resistant layer **90** and/or the anchor member **91.**

The connecting tube **80** shown in FIG. 5 extends at an angle **98** from a plane extending parallel to the center of the posterior side **88** of the housing **84,** to which the connecting tube **80** is attached. In the embodiment shown in FIG. 5, the plane is equivalently represented by the planar anchor member **91** extending from the housing **84.** The connecting tube **80** extends in a posterior direction away from the housing **84,** and away from the surface of the patient's skin **30.** The angle **98** defined by the connecting tube **80** may be at an acute angle **98** between about 20 and about 50 degrees, inclusive, preferably at approximately 30 degrees. A benefit of extending the connecting tube **80** at an angle from the housing **84** is to lessen the likelihood that the connecting tube **80** causes damage to surrounding tissue, such as tissue and viscera in the abdominal cavity below the access port **78,** after the access port **78** has been implanted subcutaneously. The connecting tube **80** extends at the angle **98** away from the housing **84,** to reduce organ erosion and/or damage caused by the presence of the tube **80.** A shallow angle of approximately 30 degrees may reduce the potential for organ erosion or damage caused by the tube **80.** In addition, the connecting tube **80** extends below the skirt of the anchor member **91,** which may serve to protect, shield, or cover, the tube **80** from errantly placed needles that do not contact the housing **84.** The anchor member **91** may serve as a shield for the tube **80.** The access port **78** shown in FIG. 5 includes an exterior layer **96,** an intermediate gel layer **92** and an interior layer **94,** similar to the embodiment shown in FIG. 2.

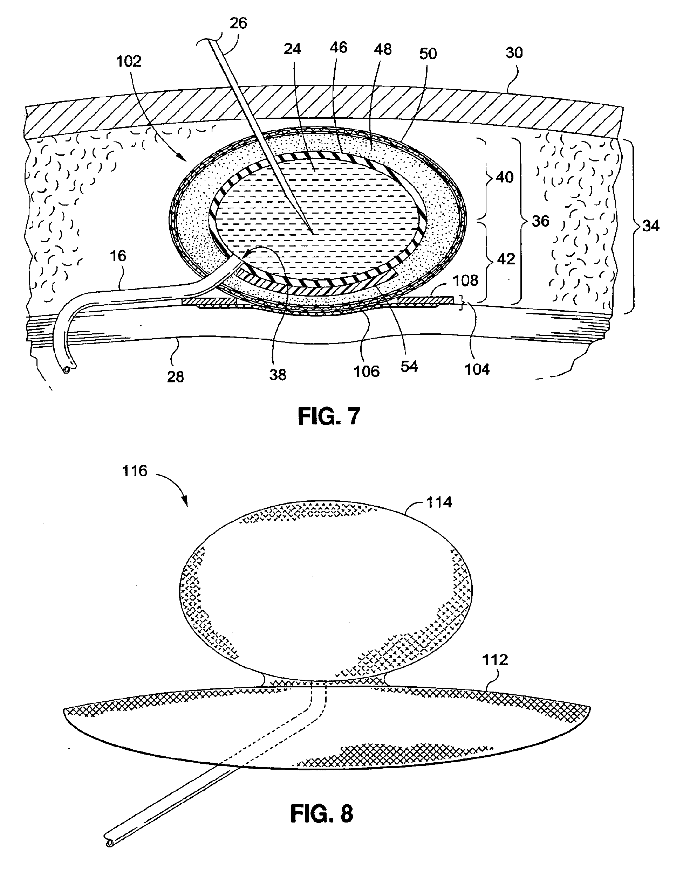

FIG. 6 illustrates an access port **102** with an anchor member **104** comprising a mesh member, or piece of mesh **106** coupled to a ring **108.** The mesh **106** may be made from a similar mesh material as the mesh anchor member **32** described in relation to FIG. 2. The mesh **106** may have a circular, square, or other symmetric shape. The mesh **106** may be fused to the ring **108,** for example, by ultrasonic welding. The ring **108** may have a rectangular or circular shape, with a substantially circular hole through the center of the ring **108.** The ring **108** may be made of a hard, needle resistant material such as a plastic, or the like. The ring **108** may have apertures **110** on the corners or periphery of the ring **108.** The apertures **110** may be configured for coupling the ring **108** to the desired portion of the patient's body. The apertures **110** may be shaped to receive sutures or other means to anchor the anchor member **104** to the desired portion of the patient's body. The mesh **106** may be circumscribed within the substantially circular hole within the ring **108,** or an outer portion of the mesh **106** may overlap part of the ring **108** and attach to the ring **108,** as shown in FIG. 7.

FIG. 7 illustrates a cross-section view of the anchor member **104** comprising the mesh **106** and the ring **108** shown in FIG. 6. The portion of the mesh **106** exposed through the circular hole of the ring **108** is bonded to the posterior side **42** of the housing **36.** The ring **108** is not bonded directly to the housing **36,** but rather connects to the patient's body, for example, through the apertures **110** shown in FIG. 6. In one embodiment, the ring **108** may also be directly bonded to the housing **36.** In one embodiment, the mesh **106** may be attached directly to the patient's body, with or without the use of the ring **108.** In this embodiment, the mesh **106** may attach to the patient's body through sutures, surgical tacks, or the like.

A benefit of the ring **108** and the mesh **106** combination is the ring **108** may protect the patient's body from further penetration by the syringe **26** needle if the needle misses the housing **36** or passes through a portion of the posterior side **42** of the housing **36** that is not protected with the layer of the needle resistant material **54.** The ring **108** may protect the portion of the patient's body to which the housing **36** is attached (e.g., the muscle wall **28)** from needle penetration in case of an errant needle stick by the physician. In addition, the ring **108** may be used in combination with the position of the conduit **100** and connecting the tube **80** shown in FIG. 5, to allow the ring **108** and the mesh **106** to provide additional protection for the tube **80** from errant needle sticks (e.g., the tube **80** will pass beneath the mesh **106,** the ring **108,** and will pass through the needle resistant layer **54).**

FIG. 8 illustrates a mesh anchor member **112** being continuous, or contiguous, with the mesh layer **114** of the housing **116.** In this embodiment, the mesh used in the anchor member **112** and the housing **116** comprises a single piece of mesh used to serve as an anchor member **112,** and to provide structure for the housing **116.** Accordingly, the material used to form the mesh anchor member **112** is the same as the material comprising the mesh layer **114** of the housing **116.** The continuous material of the mesh anchor member **112** may extend over the entire housing **116,** or may extend over only a portion of the housing **116.** If the material of the mesh anchor member **112** only extends over a portion of the housing **116,** a different mesh material may be used to cover the remainder of the housing **116,** or may be entirely excluded. The continuous mesh material connecting the mesh anchor member **112** to the housing **116** increases the total strength and durability of the access port.

### Exemplary Case Study of Gastric Band Treatment

A 41-year-old patient contemplates surgical means for the reduction of obesity. The patient with a high body mass index, for example 44 kg/m², may be classified as morbidly obese. The patient's medical history reveals no known comorbidities other than slightly elevated blood pressure. The patient's doctor considers the patient generally a good candidate for laparoscopic adjustable gastric banding.

During laparoscopic surgery, an inflatable gastric band, for example, a gastric band contemplated by the present invention, is inserted into the patient via small incisions in the abdomen, usually about 0.5-1.5 centimeters in length, using the pars flaccida technique. The inflatable gastric band is placed around the top portion of the patient's stomach, below the esophagogastric junction, creating a small pouch above the band that limits or reduces food consumption. The tubing extending from the gastric band is brought outside the abdomen. The access port, for example, an access port contemplated by the present invention, is securely connected to the gastric band. The conduit on the posterior side of the access port is joined to a stainless steel tubing connector, which is in turn connected to tubing extending from the gastric band so that the fluid introduced into the access port can be delivered through such tubing to inflate the gastric band. The access port is then placed on the rectus muscle or in another accessible subcutaneous site. The access port is then fixed in place by suturing or surgical tacking of an anchor member of the access port onto the left upper abdominal wall rectus fascia. A self-sealing, needle penetrable anterior side of the access port faces the skin, through which the syringe needle will be inserted during follow-up recalibrations of the gastric band.

In a follow-up appointment with the physician six weeks after the laparoscopic surgery, the physician locates the access port through palpation. The physician inserts a syringe needle through the skin covering the access port, and through the penetrable, self-sealing surface of the anterior side of the access port, to inject saline into a fluid reservoir in the access port. Saline fluid flows through the conduit, and the tubing connecting the access port to the gastric band. The fluid inflates and tightens the gastric band. The physician checks the appropriateness of the adjustment by having the patient drink water. If the patient is unable to swallow, the doctor removes saline fluid from the access port.

### Alternative or Supplemental Design and Construction of Access Port Housing, and Methods of Manufacture

In one embodiment of the invention, an alternative or supplemental method of making the housing is provided wherein the method generally comprises the steps of providing a plurality of mesh segments, positioning the plurality of segments on a curved molding surface, applying a fluid elastomeric material to the molding surface with the segments positioned thereon, and allowing the elastomeric material to set to form a flexible shell having an open end, the shell including the fabric segments embedded within the set elastomer, and the shell being useful as the housing of the access port. The step of positioning may include substantially entirely covering the molding surface with the mesh segments, for example, in a manner such that the mesh segments overlap one another. The method further comprises the step of sealing the open end of the elastomeric shell, for example, by providing a puncture resistant material and sealing the puncture resistant material to the open end of the elastomeric shell.

In one embodiment, the mesh segments comprise a non-stretchable mesh fabric, for example, a substantially non-expanding polyester fabric mesh. In another embodiment, the mesh segments comprise a stretchable mesh fabric.

The method may further comprise the step of applying a tacky material to the curved molding surface prior to the step of positioning the mesh. The tacky material may be a fluid elastomeric material, for example, a silicone dispersion.

In another embodiment, the method comprises pre-shaping, for example, thermoforming, a mesh element, from a two-dimensional sheet into a three dimensional "sock" having the general shape of the molding surface. The method includes positioning the pre-shaped mesh element onto the molding surface, applying a fluid elastomeric material to the molding surface with the pre-formed mesh positioned thereon, and allowing the elastomeric material to set to form a flexible shell having an open end, the shell including the preformed mesh embedded within the set elastomer, and the shell being useful as a component of the housing of the access port.

The elastomeric material may be a silicone elastomer such as a dimethyl silicone elastomer, for example, a substantially homogeneous dimethyl-diphenyl silicone elastomer. One composition that may be useful in the present invention is described in Schuessler, et al., U.S. Application Serial No. 12/179,340, filed July 24, 2008. The elastomeric material may comprise a room temperature vulcanizing (RTV) or a high temperature vulcanizing (HTV) silicone from about 0.1-95 wt %, for example, about 1-40 wt %, for example, about 30 wt %. In an exemplary embodiment, the silicone-based fluid material is a high temperature vulcanizing (HTV) platinum-cured silicone dispersion in xylene.

The mesh layer may comprise a mesh or fabric, for example, a synthetic polymer mesh or fabric, for example, a mesh or fabric made from poly(ethylene terephthalate) (PET), polypropylene (PP), polyurethane (PU), polyamide (Nylon), polyethylene (PE), any other suitable material, or combinations thereof.

In one embodiment, the exterior layer is made by dipping two or more layers of silicone-based elastomer over a conventional mandrel, followed by placement of a pre-fabricated 2 or 4-way stretchable "sock" of the mesh layer, followed by two or more dips of the silicone-based elastomer. The reinforcing "sock" is able to take the shape of the mandrel and the fabric is trapped on both sides between the elastomer layers. In this embodiment, the stretchable pre-shaped "sock" (which may form the reinforcing mesh layer 52 shown in FIG. 2A) can be relatively easily mounted on the mandrel because of its flexibility and elasticity, making it easier to manufacture a reinforced shell with the intended shape and dimensions of the mandrel. The entire assembly forming the exterior layer is heated in an oven at a temperature and time suitable to cure the silicone.

In one embodiment of the invention, the mesh layer is provided by forming a "sock" by using a cinch. Alternatively, the mesh layer is thermoformed into "sock" by placing a single sheet of suitable material, for example, a non-stretchable mesh, over a mandrel, and gathering the mesh material. The gathered mesh material is shaped, for example, thermoformed, to take on the 3-D shape of the mandrel.

Post-curing, the reinforced shell is removed from the mandrel, and another elastomeric shell (which forms the interior layer **46** shown in FIG. 2A) is placed inside the first shell (which forms the exterior layer **50** shown in FIG. 2A). The inner layer may be a typical unreinforced elastomeric shell, or alternatively may be made similarly to that described above with respect to the exterior layer. The interior layer may have the same or smaller size relative to the exterior layer. The dual-shell assembly is mounted back on a mandrel. The size of the mandrel can be the same as the one used for the inner shell fabrication or slightly larger. The latter results in a laterally stressed inner shell with potentially enhanced sealing properties.

In one embodiment, at least one of the interior layer and the exterior layer comprises an elastomeric material comprising a substantially homogenous layer of a silicone elastomer comprising a polysiloxane backbone and having a minimum mole percent of at least 10% of a substituted or pendant chemical group that sterically retards permeation of the silicone gel through the layer. More specifically, in this embodiment, the silicone elastomer is a polydimethyl siloxane and the pendant chemical group is one of a phenyl group, for example, a diphenyl group or a methyl-phenyl group, a trifluoropropyl group, and mixtures thereof. Such materials are described in detail in Schuessler, et al., U.S. Application Serial No. 12/179,340, filed on July 24, 2008. This material may make up one or more layers of the interior layer or exterior layer.

After the interior layer and exterior layer are bonded together, a cavity formed therebetween is then filled with a material, for example, a flowable material, for example, a silicone gel. This may be accomplished using any suitable means known to those of skill in the art. In one embodiment, the gel is introduced through a plug on the exterior layer. The silicone gel between the exterior and interior layer forms the intermediate layer. After filling, the assembly made up of the interior layer, the exterior layer and the intermediate layer gel layer (similar to the gel layer **48** shown in FIG. 2A), is cured, for example, by exposing the assembly to heat in an oven for a suitable length of time. The mandrel that defines the desired shape of the housing can be round or oval, with a lower or upper pole for optimal projection. Before sealing the housing with a patch, a needle guard element, such as that described and shown elsewhere herein, may be inserted and bonded to the interior layer and/or the exterior layer.

Other compositions of the shell of the housing may include a dual lumen with pre-fabricated PET reinforced-gel sheeting. Incorporation of a reinforced gel sheeting of defined thickness (demonstrated self-sealing properties) bonded to the inside of a single standard textured shell. The key for the bonding of the gel sheeting to the inside of the shell is to coat and cure both sides of the gel sheeting with dispersion in order that employing an RTV adhesive, the coated gel will bond to smooth inner surface of a vulcanized textured shell.

Other compositions of the shell of the housing may include a molded housing with an elastomeric anterior self sealing surface. This concept utilizes a mold created to the shape of the housing. Elastomers placed into these molds in a combination of durometers and wall thickness provide surfaces that are self sealing by nature. The durometer ranges from about 30A to about 80A with a thickness greater than 0.080 inches for a self sealing surface. The molding can be done using standard techniques such as casting, liquid silicone rubber molding, thermoforming, etc.

Other compositions of the shell of the housing may include a shell embedded with micro-particles of swelling agents. Embedded into the silicone shell may be hydrogel particles that swell when in contact with liquid fillers (e.g., saline). Swelling applies tension/compressive forces onto the shell. The swelling agent may be PEG or PAA, PVA, PHEMA, ethyl cellulose or salts. See, for example, Schuessler, U.S. Application Serial No. 12/543,795, filed on August 19, 2009. The mechanism of self sealing is facilitated by the swelling of the microparticles due to saline absorption from inside the implant.

Other compositions of the shell of the housing may include silicones of appropriate durometer used in the construction of self-sealing layers. A low durometer tacky silicone under tension is trapped between an interior and exterior shell layers. Or, alternating low and high durometer materials are cured under tension when forming the shell of the housing, in the relaxed state, the middle layers are under compression. As a needle is introduced and withdrawn this low durometer layer collapses around the void left by the needle and plugs the hole preventing leakage of filler liquid.

Other compositions of the shell of the housing may include a dense, flexible, closed cell foam attached to inner surface of shell. A combination of a dual shell construction encapsulating densely packed closed cell foam prevents the saline from escaping through a straight liquid channel. The compressive forces exerted by the densely packed cell layers of foam create a tortuous path for the liquid that tries to extrude to the exterior.

Other compositions of the shell of the housing may include a grafted or physical coating of hydrogel on the inside of the shell. To facilitate the self sealing effect, the implants are coated on the internal surface with a hydrogel. The hydrogel can be a polyacrylic acid based, polyvinyl alcohol based, polyhydroxyethyl methacrylate based or similar, or a combination of one or more hydrogels. The main requirements are a sufficiently high elongation at break and swell ratio.

Other compositions of the shell of the housing may include microparticles of aqueous hydrogel as filler. In this concept hydrogel particles would be placed inside the single lumen housing and these swell when the housing is filled with saline. Since the water is trapped in a hydrogel matrix, there is no availability of unbound water for leakage, after removal of the needle from the implant. This hydrogel can be a polyacrylic acid based, polyvinyl alcohol based, polyhydroxyethyl methacrylate based or similar, or a combination of one or more hydrogels. The main requirements are a sufficiently high elongation at break and swell ratio.

Other compositions of the needle resistant material may include thermoplastic films. Thin films (0.25 -1mm) of PC, PE, PP, PET, PS, Nylon and PTFE were found to resist 21g needle puncture. These sheets can be manufactured with grooves in their design to allow for movement during insertion. They are attached using adhesives to the posterior shell of the device or alternatively are encapsulated in silicone which is vulcanized to the posterior shell layer for anchorage.

Other compositions of the needle resistant material include a flexible but relatively rigid self sealing elastomer. Thermoplastic polyurethanes (TPU's) and silicones of certain durometers have a good combination of flexibility and hardness, and are not as stiff as the previously mentioned thermoplastics. They are self-sealing in specific ranges of thickness (>lmm) and durometer (>50A) which makes them an ideal candidate for a flexible self sealing needle guard. They may allow some minimal needle tip penetration, but can provide tactile feedback required to detect the needle guard and possess the flexibility to allow a smaller incision for insertion of the housing.

Other compositions of the needle resistant material include puncture resistant fabrics. The needle resistant layer can be fabricated from a pliable fabric that is puncture, cut and tear resistant. Such fabrics are seeing increasing use in the manufacture of gloves, hunting/military and medical apparel. Such a fabric is attached to the posterior of the device using adhesives or silicone encapsulation as a needle stopping component.

Other compositions of the needle resistant material include a combination of rigid and self sealing materials. The needle guard can be a combination of a self sealing elastomer with a rigid puncture resistant material such as the thermoplastics and the puncture resistant fabrics mentioned earlier. The thickness of the two layers can be controlled to develop a needle resistant layer that provides resistance to puncture, is self sealing and flexible enough such that the housing may be easily inserted through a small incision.

Other compositions of the needle resistant material include foldable flanges in a needle stop. The concept is overlapping flexible flanges made from thermoplastics, for example, polypropylene. The flanges allow a partially foldable or manipulable (for insertion and removal) needle resistant layer, and the polypropylene is impenetrable.

The various access port embodiments described throughout this application are not limited to use in a gastric band system, or for the treatment of obesity. It is contemplated the access port may be used in various other medical applications, including other medical implantable devices, including skin expanders, or drug delivery systems.

Unless otherwise indicated, all numbers expressing quantities of ingredients, components, forces, and so forth used in the specification are to be understood as being modified in all instances by the term "about." Accordingly, unless indicated to the contrary, the numerical parameters set forth in the specification are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention. No language in the specification should be construed as indicating any non-claimed element essential to the practice of the invention.

Groupings of alternative elements or embodiments of the invention disclosed herein are not to be construed as limitations. Each group member may be referred to individually or in any combination with other members of the group or other elements found herein. It is anticipated that one or more members of a group may be included in, or deleted from, a group for reasons of convenience and/or patentability.

Certain embodiments of this invention are described herein, including the best mode known to the inventors for carrying out the invention.

Specific embodiments disclosed herein may be further limited in the claims using consisting of or and consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s).

## Claims

1. An implantable access port (14, 102) for use with a gastric band (12) for the treatment of obesity, the access port comprising:
a substantially ellipsoid-shaped housing (36) defining an internal fluid reservoir (24) and a conduit (38) for the movement of fluid into and out of the internal fluid reservoir, **characterized by** at least a portion of the housing including a mesh layer (52) for enhancing the self-sealing properties of the housing (36), by constraining materials of the housing (36), to prevent fluid from leaking from the reservoir (24); and by the access port further comprising
an anchor member (32) coupled with the housing for attachment of the access port to body tissue.

2. The access port (14, 102) of claim 1, wherein the housing (36) is substantially oblate spheroid-shaped.

3. The access port (14, 102) of claim 1, wherein the housing (36) is substantially sphere-shaped.

4. The access port (14, 102) of one of the previous claims, wherein the internal fluid reservoir (24) is substantially ellipsoid-shaped.

5. The access port (14, 102) of one of the previous claims, wherein the housing (36) is defined by three orthogonal axes being an x-axis, a y-axis, and a z-axis, the housing having an outer width along the x-axis of between 1.91 cm to 4.45 cm (0.75 to 1.75 inches), inclusive, the housing having an outer width along the y-axis of between 1.91 cm to 4.45 cm (0.75 to 1.75 inches), inclusive, and the housing having an outer height along the z-axis of between 1.52 cm to 2.54 cm (0.60 to 1.00 inches), inclusive.

6. The access port (14, 102) of one of the previous claims, wherein the housing (36) is pliant.

7. The access port (14, 102) of one of the previous claims, wherein the housing (36) has an anterior side (40) and a posterior side (42), a portion of the anterior side being needle penetrable and self-sealing, a portion of the posterior side being resistant to needle penetration.

8. The access port (14, 102) of claim 7, wherein the anterior side (40) of the housing has a dome-like shape and the posterior side (42) of the housing has a dome-like shape.

9. The access port (14, 102) of claim 7 or claim 8, wherein the portion of the anterior side (40) of the housing is made from an elastomeric material.

10. The access port (14, 102) of one of claims 7 to 9, wherein the portion of the posterior side (42) of the housing is made from a fabric.

11. The access port (14, 102) of one of claims 7 to 10, wherein the anterior side (40) of the housing includes the mesh layer, and the posterior side (42) of the housing includes the mesh layer (52).

12. The access port (14, 102) of claim 11, wherein the mesh layer (52) of the anterior side of the housing is contiguous with the mesh layer (52) of the posterior side of the housing.

13. The access port (14, 102) of one of the previous claims, wherein the housing (36) includes an exterior layer (50) of elastomeric material, an intermediate layer (48) of silicone gel, and an interior layer (46) of elastomeric material, the mesh layer (52) being embedded in the exterior layer (50) of elastomeric material.

14. The access port (102) of one of the previous claims, wherein the anchor member (104) comprises a mesh member (106).

15. The access port of claim 14, wherein:
(a) the mesh layer is contiguous with the mesh member (106) ;
(b) the anchor member comprises a substantially circular piece of mesh coupled to a ring (108), and
(c) further comprising a connecting tube (80) extending from the conduit and wherein the connecting tube extends away from the housing at an angle that is between 20 to 50 degrees, inclusive.

## Patentansprüche

1. Implantierbarer Zugangsanschluss (14, 102) zur Verwendung mit einem Magenband (12) für die Behandlung von Fettleibigkeit, der Zugangsanschluss mit:
einem im Wesentlichen ellipsoidförmigen Gehäuse (36), das ein inneres Fluidreservoir (24) und einen Kanal (38) für die Bewegung von Fluid in und aus dem inneren Fluidreservoir definiert, **dadurch gekennzeichnet, dass** zumindest ein Abschnitt des Gehäuses eine Gewebelage (52) zum Verbessern der selbstdichtenden Eigenschaften des Gehäuses (36) durch Aufhaltematerialien des Gehäuses (36) aufweist, um einem Austreten von Fluid aus dem Reservoir (24) vorzubeugen; und dass der Zugangsanschluss ferner aufweist:
ein Verankerungselement (32), das für ein Anbringen des Zugangsanschlusses an Körpergewebe mit dem Gehäuse gekoppelt ist.

2. Zugangsanschluss (14, 102) nach Anspruch 1, bei dem das Gehäuse (36) im Wesentlichen eine abgeflachte Ellipsoidform aufweist.

3. Zugangsanschluss (14, 102) nach Anspruch 1, bei dem das Gehäuse (36) im Wesentlichen eine Kugelform aufweist.

4. Zugangsanschluss (14, 102) nach einem der vorstehenden Ansprüche, bei dem das interne Fluidreservoir (24) im Wesentlichen eine Ellipsoidform aufweist.

5. Zugangsanschluss (14, 102) nach einem der vorstehenden Ansprüche, bei dem das Gehäuse (36) durch drei senkrechte Achsen definiert ist, die eine x-Achse, eine y-Achse und eine z-Achse sind, wobei das Gehäuse eine äußere Breite entlang der x-Achse von zwischen einschließlich 1,91 cm bis 4,45 cm (0,75 bis 1,75 Inch) aufweist, das Gehäuse eine äußere Breite entlang der y-Achse zwischen einschließlich 1,91 cm bis 4,45 cm (0,75 bis 1,75 Inch) aufweist und das Gehäuse eine äußere Höhe entlang der z-Achse zwischen einschließlich 1,52 cm bis 2,54 cm (0,60 bis 1,00 Inch) aufweist.

6. Zugangsanschluss (14, 102) nach einem der vorstehenden Ansprüche, bei dem das Gehäuse (36) biegsam ist.

7. Zugangsanschluss (14, 102) nach einem der vorstehenden Ansprüche, bei dem das Gehäuse (36) eine anteriore Seite (40) und eine posteriore Seite (42) aufweist, wobei ein Abschnitt der anterioren Seite mit einer Nadel durchdringbar und selbstdichtend ist und ein Abschnitt der posterioren Seite gegen ein Eindringen einer Nadel resistent ist.

8. Zugangsanschluss (14, 102) nach Anspruch 7, bei dem die anteriore Seite (40) des Gehäuses eine kuppelähnliche Form aufweist und die posteriore Seite (42) des Gehäuses eine kuppelähnliche Form aufweist.

9. Zugangsanschluss (14, 102) nach Anspruch 7 oder 8, bei dem der Abschnitt der anterioren Seite (40) des Gehäuses aus einem Elastomermaterial hergestellt ist.

10. Zugangsanschluss (14, 102) nach einem der Ansprüche 7 bis 9, bei dem der Abschnitt der posterioren Seite (42) des Gehäuses aus einem Stoff hergestellt ist.

11. Zugangsanschluss (14, 102) nach einem der Ansprüche 7 bis 10, bei dem die anteriore Seite (40) des Gehäuses die Gewebelage aufweist und die posteriore Seite (42) des Gehäuses die Gewebelage (52) aufweist.

12. Zugangsanschluss (14, 102) nach Anspruch 11, bei dem die Gewebelage (52) der anterioren Seite des Gehäuses mit der Gewebelage (52) der posterioren Seite des Gehäuses zusammenhängend ist.

13. Zugangsanschluss (14, 102) nach einem der vorstehenden Ansprüche, bei dem das Gehäuse (36) eine äußere Lage (50) aus Elastomermaterial, eine Zwischenlage (48) aus Silikongel und eine innere Lage (46) aus Elastomermaterial aufweist, wobei die Gewebelage (52) in der äußeren Lage (50) aus Elastomermaterial eingebettet ist.

14. Zugangsanschluss (102) nach einem der vorstehenden Ansprüche, bei dem das Verankerungselement (104) ein Gewebeelement (106) aufweist.

15. Zugangsanschluss nach Anspruch 14, bei dem:
(a) die Gewebelage mit der Gewebelage (106) zusammenhängend ist;
(b) das Verankerungselement ein im Wesentlichen kreisförmiges Gewebestück aufweist, das mit einem Ring (108) gekoppelt ist, und
(c) ferner mit einem Verbindungsrohr (80), das sich von dem Kanal erstreckt und wobei das Verbindungsrohr sich in einem Winkel weg von dem Gehäuse erstreckt, der zwischen einschließlich 20° und 50° liegt.

## Revendications

1. Orifice d'accès implantable (14, 102) pour une utilisation avec un anneau gastrique (12) pour le traitement de l'obésité, l'orifice d'accès comprenant :
un boîtier en forme substantiellement ellipsoïde (36) définissant un réservoir de fluide interne (24) et un conduit (38) pour le déplacement de fluide dans le réservoir de fluide interne et en dehors de celui-ci, **caractérisé par** au moins une partie du boîtier comportant une couche de maille (52) pour améliorer les propriétés d'auto-obturation du boîtier (36), par des matériaux de contrainte du boîtier (36), pour empêcher un fluide de s'écouler depuis le réservoir (24) ; et par l'orifice d'accès comprenant en outre
un élément d'ancrage (32) couplé au boîtier pour la fixation de l'orifice d'accès au tissu corporel.

2. Orifice d'accès (14, 102) de la revendication 1, dans lequel le boîtier (36) est de forme substantiellement sphéroïde aplatie.

3. Orifice d'accès (14, 102) de la revendication 1, dans lequel le boîtier (36) est de forme substantiellement sphérique.

4. Orifice d'accès (14, 102) de l'une des revendications précédentes, dans lequel le réservoir de fluide interne (24) est de forme substantiellement ellipsoïde.

5. Orifice d'accès (14, 102) de l'une des revendications précédentes, dans lequel le boîtier (36) est défini par trois axes orthogonaux qui sont un axe x, un axe y et un axe z, le boîtier ayant une largeur externe le long de l'axe x comprise entre 1,91 cm et 4,45 cm (0,75 et 1,75 pouces), inclus, le boîtier ayant une largeur externe le long de l'axe y comprise entre 1,91 cm et 4,45 cm (0,75 et 1,75 pouces), inclus, et le boîtier ayant une hauteur externe le long de l'axe z comprise entre 1,52 cm et 2,54 cm (0,60 et 1,00 pouces), inclus.

6. Orifice d'accès (14, 102) de l'une des revendications précédentes, dans lequel le boîtier (36) est pliable.

7. Orifice d'accès (14, 102) de l'une des revendications précédentes, dans lequel le boîtier (36) a un côté antérieur (40) et un côté postérieur (42), une partie du côté antérieur étant pénétrable par l'aiguille et auto-obturante, une partie du côté postérieur étant résistante à la pénétration de l'aiguille.

8. Orifice d'accès (14, 102) de la revendication 7, dans lequel le côté antérieur (40) du boîtier a une forme de dôme et le côté postérieur (42) du boîtier a une forme de dôme.

9. Orifice d'accès (14, 102) de la revendication 7 ou 8, dans lequel la partie du côté antérieur (40) du boîtier est réalisée en un matériau élastomère.

10. Orifice d'accès (14, 102) de l'une des revendications 7 à 9, dans lequel la partie du côté postérieur (42) du boîtier est réalisée en un tissu.

11. Orifice d'accès (14, 102) de l'une des revendications 7 à 10, dans lequel le côté antérieur (40) du boîtier comporte la couche de maille, et le côté postérieur (42) du boîtier comporte la couche de maille (52).

12. Orifice d'accès (14, 102) de la revendication 11, dans lequel la couche de maille (52) du côté antérieur du boîtier est contiguë à la couche de maille (52) du côté postérieur du boîtier.

13. Orifice d'accès (14, 102) de l'une des revendications précédentes, dans lequel le boîtier (36) comporte une couche externe (50) de matériau élastomère, une couche intermédiaire (48) de gel de silicone, et une couche interne (46) de matériau élastomère, la couche de maille (52) étant intégrée dans la couche externe (50) de matériau élastomère.

14. Orifice d'accès (102) de l'une des revendications précédentes, dans lequel l'élément d'ancrage (104) comprend un élément à mailles (106).

15. Orifice d'accès de la revendication 14, dans lequel :
(a) la couche de maille est contiguë à l'élément à mailles (106) ;
(b) l'élément d'ancrage comprend un morceau substantiellement circulaire de maille couplée à un anneau (108), et
(c) comprenant en outre un tube de raccordement (80) s'étendant depuis le conduit et dans lequel le tube de raccordement s'étend loin du boîtier à un angle qui est compris entre 20 et 50 degrés, inclus.
